# EUROPÄISCHE PATENTSCHRIFT

(11) **EP 1 682 471 B1**
(45) Veröffentlichungstag und Bekanntmachung des Hinweises auf die Patenterteilung: **20.01.2010**
(21) Anmeldenummer: 04797622.0
(22) Anmeldetag: 04.11.2004
(51) Int. Cl.: C07C 23/16, C07C 69/14, C07C 43/184, C08F 10/10, C08F 110/10

(54) **SUBSTITUIERTE CYCLOALKANE UND IHRE VERWENDUNG ALS INITIATOREN ZUR KATIONISCHEN POLYMERISATION**
SUBSTITUTED CYCLOALKANES, AND USE THEREOF AS CATIONIC POLYMERIZATION INITIATORS
CYCLOALCANES SUBSTITUES ET LEUR UTILISATION COMME INITIATEURS POUR LA POLYMERISATION CATIONIQUE

(30) Priorität: 05.11.2003 DE 10351643
(43) Veröffentlichungstag der Anmeldung: 26.07.2006
(73) Patentinhaber: BASF SE, 67056 Ludwigshafen (DE); Ludwig-Maximilians-Universität München, 80359 München (DE)
(72) Erfinder: MIJOLOVIC, Darijo, 68309 Mannheim (DE); HERRLICH-LOOS, Mirjam, 68165 Mannheim (DE); LANG, Gabriele, 68167 Mannheim (DE); MAYR, Herbert, 82319 Starnberg (DE)
(86) Internationale Anmeldenummer: PCT/EP2004/012498
(87) Internationale Veröffentlichungsnummer: WO 2005/044766

(56) Entgegenhaltungen:
- EP-A- 0 713 883
- WO-A-02/096964

## Beschreibung

Die vorliegende Erfindung betrifft substituierte Cycloalkane, die Abgangsgruppen an tertiären Ringkohlenstoffatomen aufweisen, ein Verfahren zu ihrer Herstellung und ihre Verwendung als Initiatoren zur kationischen Polymerisation, insbesondere zur kationischen Polymerisation von Isobuten.

Bei der Herstellung von Isobutenpolymeren durch kationische Polymerisation (auch als "lebende kationische Polymerisation" bezeichnet) werden Initiatorsysteme eingesetzt, die eine Lewis-Säure und eine organische Verbindung umfassen, die mit der Lewis-Säure ein Carbokation oder einen kationogenen Komplex bildet.

Für die Weiterverarbeitung, beispielsweise zu Dichtungs- und Versiegelungsmassen oder zu Klebe(roh)stoffen, besonders geeignete Isobutenpolymere sind telechel, d.h. sie weisen zwei oder mehr reaktive Endgruppen auf. Bei diesen Endgruppen handelt es sich vor allem um Kohlenstoff-Kohlenstoff-Doppelbindungen, die sich weiter funktionalisieren lassen, oder um mit einem Terminierungsagens funktionalisierte Gruppen. So beschreibt die EP-A 713 883 die Herstellung telecheler Isobuten-Polymere unter Verwendung eines mindestens difunktionellen Initiators, wie Dicumylchlorid. Nachteilig an dem bekannten Verfahren ist, dass die beschriebenen aromatischen Initiatoren zu I ndanyl- oder Diindangruppen reagieren können (vgl. Cr. Pratrap, S.A. Mustafa, J.P. Heller, J. Polym. Sci. Part A, Polym. Chem. 1993, 31, S. 2387-2391), was die Synthese definierter telecheler Isobutenpolymere beeinträchtigt.

Es besteht ein ständiger Bedarf nach neuen Initiatoren für die kationische Polymerisation. Aufgabe der vorliegenden Erfindung war daher, neue Initiatoren für die kationische Polymerisation, insbesondere die kationische Polymerisation von Isobuten, bereitzustellen. Die Initiatoren sollen die zuvor dargestellten Nachteile nicht aufweisen, leicht herstellbar und lagerstabil sein.

Die Aufgabe wird durch ein substituiertes Cycloalkan gelöst, das einen 7- bis 12-gliedrigen carbocyclischen Ring der Formel I umfasst 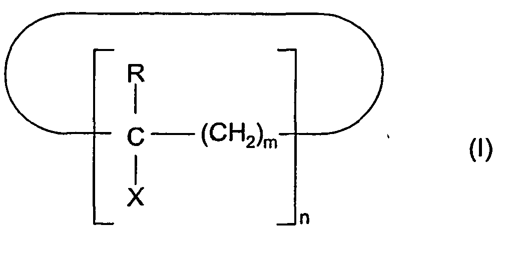 worin
R für C₁-C₆-Alkyl steht,
X für Halogen, OR¹ oder für OCOR' steht, worin R¹ für C₁-C₆-Alkyl steht,
n für 2 oder 3 steht und
m für eine ganze Zahl mit einem Wert von wenigstens 1, vorzugsweise wenigstens 2, steht.

X, R und m können in jeder Wiederholungseinheit verschiedene Bedeutungen haben. Vorzugsweise haben X und R in jeder Wiederholungseinheit die gleiche Bedeutung.

Der Ausdruck C₁-C₆-Alkyl steht für lineare oder verzweigte Alkylgruppen mit 1 bis 6 Kohlenstoffatomen, wie Methyl, Ethyl, n-Propyl, Isopropyl, n-Butyl, sec-Butyl, Isobutyl, tert-Butyl, n-Pentyl, 2-Methylbutyl, Neopentyl, n-Hexyl, 2-Methylpentyl, 3-Methylpentyl, 2,2-Dimethylbutyl, 2,3-Dimethylbutyl oder 2-Ethylbutyl.

Halogen steht vorzugsweise für Chlor, Brom oder Iod, besonders bevorzugt für Chlor oder Brom und speziell für Chlor.

Bevorzugt steht R für C₁-C₄-Alkyl, vorzugsweise Methyl oder Ethyl, insbesondere Methyl.

X steht vorzugsweise für Halogen; Acetyloxy oder Propionyloxy; Methoxy, Ethoxy, Propoxy oder Butoxy, besonders bevorzugt für Halogen und insbesondere für Chlor.

Bei den Cycloalkanen der Formel I handelt es sich um 7- bis 12-gliedrige carbocylische Ringe, vorzugsweise 7-, 8- oder 9- gliedrige carbocyclische Ringe. Besonders bevorzugt sind 8-gliedrige Ringe.

Die tertiären Kohlenstoffatome, die die Gruppen X tragen, sind durch wenigstens eine Methylengruppe, vorzugsweise wenigstens 2 Methylengruppen getrennt.

Bevorzugte Cycloalkane der Formel I sind solche der folgenden Formeln Ia bis Ic, worin
o = 1 und p = 2, oder
o = 1 oder 2, p = 2 oder 3 und o+p = 4, oder
o = 1 oder 2, p = 3 oder 4 und o+p = 5:

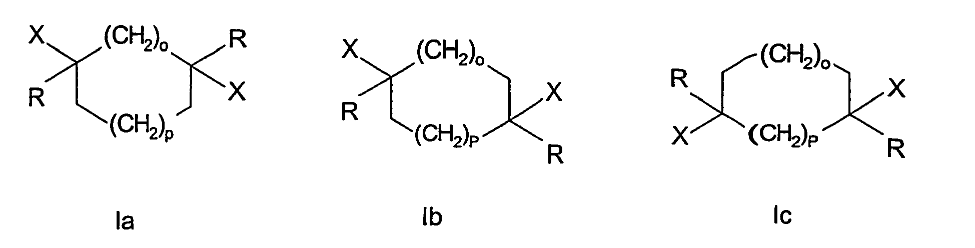

Besonders bevorzugte substituierte Cycloalkane der Formel I sind 1,5-Dichlor-1,5-dimethylcyclooctan und 1,4-Dichlor-1,4-dimethylcyclooctan der folgenden Formeln Id bzw. le, worin R für Methyl und X für CI steht.

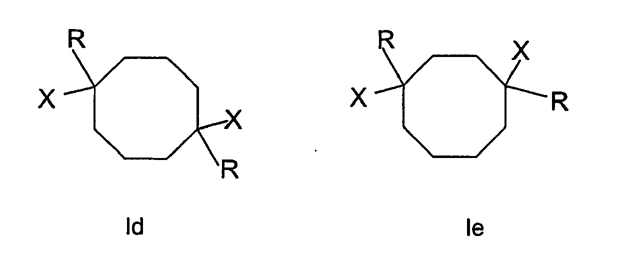

Die erfindungsgemäßen substituierten Cycloalkane der Formel I sind auf verschiedene Weise erhältlich, beispielsweise durch Addition einer Verbindung HX an Cycloalkadiene oder-triene, die an den Doppelbindungen jeweils einen C₁-C₆-Alkylsubstituenten tragen. Die primären Additionsprodukte können gegebenenfalls weiter derivatisiert werden.

Ein weiterer Gegenstand der vorliegenden Erfindung ist daher ein Verfahren zur Herstellung eines substituieren Cycloalkans der Formel I, bei dem man ein Cycloalkapolyen der Formel II 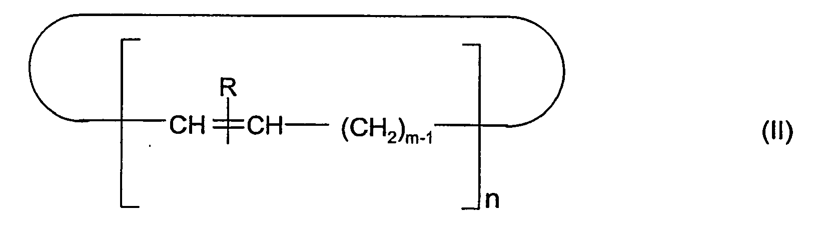 bei einer Temperatur von weniger als 40 °C mit einer Verbindung HX umsetzt, wobei die Symbole R, X, m und n die bereits angegebene Bedeutung haben.

Das 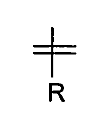 Symbol
bedeutet, dass eines der beiden Wasserstoffatome an der C=C-Doppelbindung durch "R" substituiert ist.

Bevorzugt weisen in den Cycloalkapolyenen der Formel II alle Doppelbindungen cis-Konfiguration auf. Die Doppelbindungen sind vorzugsweise nicht kumuliert und vorzugsweise nicht konjugiert.

Bevorzugte Cycloalkapolyene sind solche der folgenden Formeln IIa bis IIc, worin
o = 1 und p = 2, oder
o = 1 oder 2, p = 2 oder 3 und o+p = 4, oder
o = 1 oder 2, p = 3 oder 4 und o+p = 5.

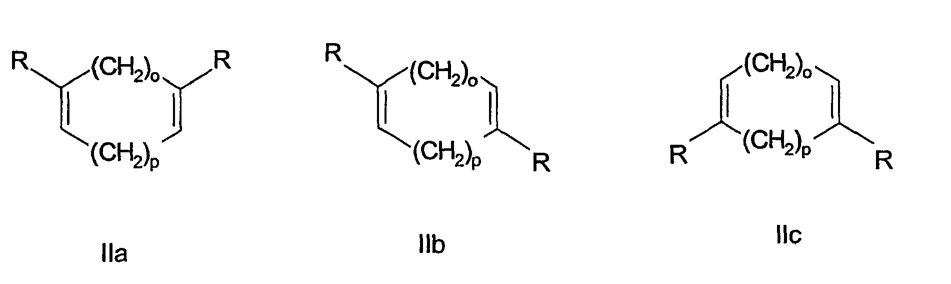

Besonders bevorzugte Cycloalkapolyene der Formel II sind 1,5-Dialkylcycloocta-1,5-diene oder 1,6-Dialkylcycloocta-1,5-diene der Formeln IId und IIe:

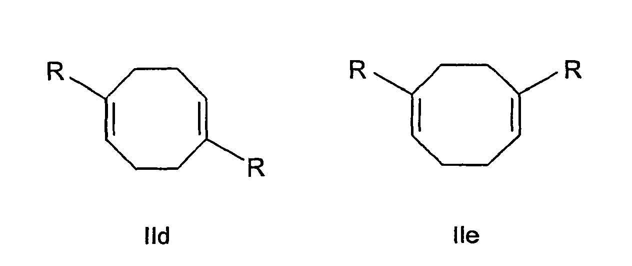

Am meisten bevorzugt sind 1,5-Dimethylcycloocta-1,5-dien oder 1,6-Dimethylcyclo-octa-1,5-dien.

Man kann auch Gemische von Cycloalkapolyenen der Formel II einsetzen, vorzugsweise solcher gleicher Ringgröße und gleicher Anzahl an Doppelbindungen. Insbesondere unterscheiden sich die enthaltenen Verbindungen lediglich durch die Position der Substituenten R an den Doppelbindungen. Speziell verwendet man 1,5-Dimethylcyclo-octa-1,5-dien oder 1,6-Dimethylcycloocta-1,5-dien oder Gemische davon.

Cycloalkapolyene der Formel II und Verfahren zu ihrer Herstellung sind bekannt. Beispielhaft sei die Herstellung von 1,5-Dimethylcycloocta-1,5-dien oder 1,6-Dimethyl-cycloocta-1,5-dien durch Dimerisierung von Isopren genannt (vgl. G. A. Tolstikov et al., J. Gen. Chem. USSR (Engl. Transl.); 46, 1976, S. 188-192; G. S. Hammond et al., J. Org. Chem., 28, 1963, S. 3297-3303).

Bei der Verbindung HX handelt es sich zweckmäßigerweise um einen Halogenwasserstoff oder um eine organische Carbonsäure R¹COOH. Beispiele für geeignete Halogenwasserstoffe sind Chlorwasserstoff, Bromwasserstoff und Iodwasserstoff. Beispiele für geeignete organische Carbonsäuren R¹COOH sind Ameisensäure, Essigsäure, Propionsäure, Buttersäure, Isobuttersäure, Valeriansäure, Isovaleriansäure und Capronsäure.

Vorzugsweise verwendet man als Verbindung HX einen Halogenwasserstoff, insbesondere Chlorwasserstoff. Der Halogenwasserstoff wird vorzugsweise gasförmig oder in Form einer Lösung in einem organischen Lösungsmittel eingesetzt, z. B. eine etherische Lösung von Chlorwasserstoff- oder Bromwasserstoff in aliphatischen oder cyclischen Ethern, wie Diethylether, Methyl-tert-butylether, Propylether, Isopropylether, Tetrahydrofuran und Dioxan. Besonders bevorzugt ist jedoch die Verwendung gasförmiger Halogenwasserstoffe, insbesondere gasförmigem Chlorwasserstoff.

Die Verbindung HX wird in wenigstens stöchiometrischer Menge, bezogen auf die im Cycloalkapolyen enthaltenen Doppelbindungen verwendet, vorzugsweise in einem 1,1-bis 10-fachen molaren Überschuss.

Die Umsetzung erfolgt im Allgemeinen bei einer Temperatur von - 25 bis + 25 °C, vorzugsweise - 5 bis +10 °C. Die Umsetzung kann sowohl bei Normaldruck als auch bei Überdruck erfolgen. Vorzugsweise beträgt der Druck 1 bis 10 bar.

Die Umsetzung kann in Gegenwart eines Lösungsmittels erfolgen. Es kommen alle Lösungsmittel oder deren Gemische in Betracht, die eine geeignete Dielektrizitätskonstante und keine abstrahierbaren Protonen aufweisen und die unter den Reaktionsbedingungen flüssig sind. Beispiele hierfür sind aliphatische Kohlenwasserstoffe, z.B. Alkane mit 4 bis 8, vorzugsweise 5 bis 8 Kohlenstoffatomen, wie Butan, Pentan, Hexan, Heptan, Octan und deren Isomere, Halogenalkane, wie Methylchlorid, Methylbromid, Methylenchlorid, Methylenbromid, Trichlormethan, Tetrachlorkohlenstoff, Chlorethan, Dichlorethan und Trichlorethan, Cycloalkane mit 5 bis 8 Kohlenstofatomen, wie Cyclopentan, Cyclohexan und Cyclooctan, außerdem aromatische Kohlenwasserstoffe, wie Benzol, Toluol, die Xylole, Ethylbenzol, Nitrobenzol, Chlorbenzol und Dichlorbenzol.

Vorzugsweise erfolgt die Umsetzung jedoch in Abwesenheit eines Lösungsmittels.

Gewünschtenfalls kann die Umsetzung des Cycloalkapolyens der Formel II mit der Verbindung HX auch in Gegenwart eines Katalysators, wie Lewis- und/oder Brönsted-Säuren, erfolgen.

Geeignete Lewis-Säuren sind Aluminiumchlorid, Bortrifluorid, Bortrifluoridalkoholat oder -etherat, Bortrichlorid, Titantetrachlorid und Zinntetrachlorid.

Geeignete Brönsted-Säuren sind solche mit größerer Säurestärke als die Verbindung HX. Bei der Brönsted-Säure kann es sich sowohl um eine anorganische Säure handeln, wie Schwefelsäure, Phosphorsäure oder Iodwasserstoff, oder um eine starke organische Säure, wie Trifluoressigsäure oder Trifluormethansulfonsäure. Die organische Säure kann auch in gebundener Form vorliegen, z. B. als Ionenaustauscherharz.

Die Umsetzung des Cycloalkapolyens der Formel II und der Verbindung HX kann nach üblichen Verfahren erfolgen. So kann man beispielsweise das Cycloalkapolyen, gegebenenfalls in einem Lösungsmittel und gegebenenfalls gemeinsam mit einem Katalysator, bei der Reaktionstemperatur vorlegen und die Verbindung HX zugeben. Die Zugabe der Verbindung HX erfolgt je nach Natur dieser Verbindung. So können gasförmig eingesetzte Halogenwasserstoffe durch das vorgelegte Edukt oder durch dessen Lösung hindurchgeleitet werden. Alternativ kann in einem Druckgefäß gearbeitet werden, in welches die Gesamtmenge des einzusetzenden Halogenwasserstoffs eingeleitet wird und das Reaktionsgemisch für die Dauer der erforderlichen Reaktionszeit belassen oder gemischt wird. Alternativ kann der Halogenwasserstoff auch nach und nach, gegebenenfalls nach Maßgabe des Verbrauchs, in das Druckgefäß eingeleitet werden. Bei Verwendung von Halogenwasserstoff-Lösungen können diese auf einmal oder bevorzugt portionsweise oder kontinuierlich zu dem Cycloalkapolyenen der Formel II bzw. ihrer Lösung gegeben werden. Organische Carbonsäuren HX können als solche oder in Lösung eingesetzt werden.

Die Aufarbeitung erfolgt nach üblichen Verfahren. So kann überschüssiger Halogenwasserstoff beispielsweise durch Strippen mit einem Inertgas, wie Stickstoff, oder destillativ, z. B. unter vermindertem Druck, entfernt werden. Wurde die Umsetzung lösungsmittelfrei und ohne Katalysator durchgeführt, so erübrigt sich häufig eine weitere Aufreinigung des Produkts. Bei Umsetzung in Lösung wird in der Regel das Lösungsmittel nach Entfernen des Halogenwasserstoffs entfernt, was beispielsweise destillativ erfolgen kann. Bei Verwendung von Säuren R¹COOH als Verbindung HX oder bei Umsetzung in Gegenwart von Lewis- oder Brönsted-Säuren werden diese meist extraktiv, z. B. durch Extraktion des Reaktionsgemischs mit Wasser oder einer wässrigen Base, entfernt.

Das Produkt kann anschließend mittels üblicher Verfahren, beispielsweise destillativ, insbesondere unter vermindertem Druck, aufgereinigt werden. Mit dem erfindungsgemäßen Verfahren ist das Reaktionsprodukt jedoch häufig auch ohne Aufreinigung in einem Reinheitsgrad erhältlich, der für weitere Anwendungen ausreicht.

Verbindungen I, in denen X für einen Rest OR¹ steht, können außerdem erhalten werden, indem man ein substituiertes Cycloalkan der Formel I, worin X für Halogen steht, unter dem Fachmann bekannten Bedingungen einer nukleophilen Substitution mit einem Alkohol R¹OH umsetzt.

Die Erfindung betrifft außerdem ein Verfahren der kationischen Polymerisation, bei dem man kationisch polymerisierbare ethylenisch ungesättigte Monomere in Gegenwart eines substituierten Cycloalkans der Formel I und einer Lewis-Säure polymerisiert. Es werden je nach substituiertem Cycloalkan der Formel I lineare (mit n = 2) oder sternförmige (mit n = 3) Polymere erhalten.

Als kationisch polymerisierbare ethylenisch ungesättigte Monomere kommen vor allem Isobuten, vinylaromatischeVerbindungen wie Styrol und α-Methylstyrol, oder Isoolefine mit 5 bis 10 C-Atomen wie 2-Methylbuten-1, 2-Methylpenten-1, 2-Methylhexen-1, 2-Ethylpenten-1, 2-Ethylhexen-1 und 2-Propylhepten-1 in Betracht. Vorzugsweise dient das Verfahren der Herstellung von Homo-, Co- oder Blockcopolymeren des Isobutens.

Als Lewis-Säure kommen kovalente Metallhalogenide und Halbmetallhalogenide, die eine Elektronenpaarlücke aufweisen, in Betracht. Derartige Verbindungen sind dem Fachmann bekannt, beispielsweise aus J.P. Kennedy et al. in US 4,946,889, US 4,327,201, US 5,169,914, EP-A-206 756, EP-A-265 053 sowie umfassend in J.P. Kennedy, B. Ivan, "Designed Polymers by Carbocationic Macromolecular Engineering", Oxford University Press, New York, 1991. Für die Isobuten-Polymerisation besonders bevorzugte Lewis-Säuren sind Titantetrachlorid, Bortrichlorid und Bortrifluorid und insbesondere Titantetrachlorid.

Es hat sich bewährt, die Polymerisation in Gegenwart eines Elektronendonors durchzuführen. Als Elektronendonoren kommen aprotische organische Verbindungen in Betracht, die ein an einem Stickstoff, Sauerstoff oder Schwefelatom befindliches, freies Elektronenpaar aufweisen. Bevorzugte Donorverbindungen sind ausgewählt unter Pyridinen wie Pyridin selbst, 2,6-Dimethylpyridin, sowie sterisch gehinderten Pyridinen wie 2,6-Diisopropylpyridin und 2,6-Di-tert-butylpyridin; Amiden, insbesondere N,N-Dialkylamiden von aliphatischen oder aromatischen Carbonsäuren wie N,N-Dimethylacetamid; Lactamen, insbesondere N-Alkyllactamen wie N-Methylpyrrolidon; Ethern, z.B. Dialkylethern wie Diethylether und Diisopropylether, cyclischen Ethern, wie Tetrahydrofuran; Aminen, insbesondere Trialkylaminen wie Triethylamin; Estern, insbesondere C₁-C₄-Alkylestern aliphatischer C₁-C₆-Carbonsäuren wie Ethylacetat; Thioethern, insbesondere Dialkylthioethern oder Alkylarylthioethern, wie Methylphenylsulfid; Sulfoxiden, insbesondere Dialkylsulfoxiden, wie Dimethylsulfoxid; Nitrilen, insbesondere Alkylnitrilen wie Acetonitril und Propionitril; Phosphinen, insbesondere Trialkylphosphinen oder Triarylphosphinen, wie Trimethylphosphin, Triethylphosphin, Tri-n-butylphosphin und Triphenylphosphin und nicht polymerisierbaren, aprotischen siliziumorganischen Verbindungen, die wenigstens einen über Sauerstoff gebundenen organischen Rest aufweisen.

Unter den vorgenannten Donoren sind Pyridin und sterisch gehinderte Pyridin-Derivate sowie insbesondere siliziumorganische Verbindungen bevorzugt. Beispiele für derartige bevorzugte Verbindungen sind Dimethoxydiisopropylsilan, Dimethoxyisobutylisopropylsilan, Dimethoxydi-isobutylsilan, Dimethoxydicyclopentylsilan, Dimethoxyiso-butyl-2-butylsilan, Diethoxyisobutylisopropylsilan, Triethoxytoluylsilan, Triethoxybenzylsilan und Triethoxyphenylsilan.

Die Lewis-Säure wird in einer Menge eingesetzt, die zur Bildung des Initiatorkomplexes ausreicht. Das Molverhältnis von Lewis-Säure zu Initiator beträgt im Allgemeinen 10 n : 1 bis 1 n : 1, insbesondere 2,5 n bis 1 n : 1, wobei n für die Funktionalität des Initiators steht.

Als Isobuten-Einsatzstoffe eignen sich sowohl Isobuten selbst als auch isobutenhaltige C₄-Kohlenwasserstoffströme, beispielsweise C₄-Raffinate, C₄-Schnitte aus der Isobutan-Dehydrierung, C₄-Schnitte aus Steamcrackem, FCC-Crackern (FCC: Fluid Catalyzed Cracking), sofern sie weitgehend von darin enthaltenem 1,3-Butadien befreit sind. Erfindungsgemäß geeignete C₄-Kohlenwasserstoffströme enthalten in der Regel weniger als 500 ppm, vorzugsweise weniger als 200 ppm Butadien. Bei Einsatz von C₄-Schnitten als Einsatzmaterial übernehmen die von Isobuten verschiedenen Kohlenwasserstoffe die Rolle eines inerten Lösungsmittels.

Es können auch Monomermischungen des Isobutens mit olefinisch ungesättigten Monomeren, welche mit Isobuten unter kationischen Polymerisationsbedingungen copolymerisierbar sind, umgesetzt werden. Das erfindungsgemäße Verfahren ist außerdem zur Blockcopolymerisation von Isobuten mit unter kationischen Polymerisationsbedingungen polymerisierbaren ethylenisch ungesättigten Comonomeren geeignet. Sofern Monomermischungen des Isobutens mit geeigneten Comonomeren polymerisiert werden sollen, enthält die Monomermischung vorzugsweise mehr als 80 Gew.%, insbesondere mehr als 90 Gew.-%, und, besonders bevorzugt, mehr als 95 Gew.-% Isobuten, und weniger als 20 Gew.-%, vorzugsweise weniger als 10 Gew.-%, und insbesondere weniger als 5 Gew.-%, Comonomere.

Als copolymerisierbare Monomere kommen Vinylaromaten wie Styrol und C₁-C₄-Alkylstyrole wie 2-, 3- und 4-Methylstyrol, sowie 4-tert.-Butylstyrol, n-Buten, Isoolefine mit 5 bis 10 C-Atomen wie 2-Methylbuten-1, 2-Methylpenten-1, 2-Methylhexen-1, 2-Ethylpenten-1, 2-Ethylhexen-1 und 2-Propylhepten-1 in Betracht. Als Comonomere kommen weiterhin Olefine in Betracht, die eine Silylgruppe aufweisen wie 1-Trimethoxysilylethen, 1-(Trimethoxysilyl)propen, 1-(Trimethoxysilyl)-2-methylpropen-2, 1-(Tri(methoxyethoxy)si-lyl]ethen, 1-[Tri(methoxyethoxy)silyl]propen, und 1-[Tri(methoxyethoxy)silyl]-2-methylpropen-2.

Die Polymerisation wird üblicherweise in einem Lösungsmittel durchgeführt. Als Lösungsmittel kommen alle niedermolekularen, organischen Verbindungen oder deren Gemische in Betracht, die eine geeignete Dielektrizitätskonstante und keine abstrahierbaren Protonen aufweisen und die unter den Polymerisationsbedingungen flüssig sind. Bevorzugte Lösungsmittel sind Kohlenwasserstoffe, z.B. acyclische Kohlenwasserstoffe mit 2 bis 8 und vorzugsweise 3 bis 8 Kohlenstoffatomen wie Ethan, Iso- und n-Propan, n-Butan und seine Isomeren, n-Pentan und seine Isomeren, n-Hexan und seine Isomeren, sowie n-Heptan und seine Isomeren, sowie n-Octan und seine Isomeren, cyclische Alkane mit 5 bis 8 Kohlenstoffatomen wie Cyclopentan, Methylcyclopentan, Cyclohexan, Methylcyclohexan, Cycloheptan, acyclische Alkene mit vorzugsweise 2 bis 8 Kohlenstoffatomen wie Ethen, Iso- und n-Propen, n-Buten, n-Penten, n-Hexen und n-Hepten, cyclische Olefine wie Cyclopenten, Cyclohexen und Cyclohepten, aromatische Kohlenwasserstoffe wie Toluol, Xylol, Ethylbenzol, sowie halogenierte Kohlenwasserstoffe, wie halogenierte aliphatische Kohlenwasserstoffe, z.B. wie Chlormethan, Dichlormethan, Trichlormethan, Chlorethan, 1,2-Dichlorethan und 1,1,1-Trichlorethan und 1-Chlorbutan, sowie halogenierte aromatische Kohlenwasserstoffe wie Chlorbenzol und Fluorbenzol. Die als Lösungsmittel verwendeten halogenierten Kohlenwasserstoffe umfassen keine Verbindungen, worin Halogenatome an sekundären oder tertiären Kohlenstoffatomen sitzen.

Besonders bevorzugte Lösungsmittel sind aromatische Kohlenwasserstoffe, wovon Toluol besonders bevorzugt ist. Ebenfalls bevorzugt sind Lösungsmittelgemische, die wenigstens einen halogenierten Kohlenwasserstoff und wenigstens einen aliphatischen oder aromatischen Kohlenwasserstoff umfassen. Insbesondere umfasst das Lösungsmittelgemisch Hexan und Chlormethan und/oder Dichlormethan. Das Volumenverhältnis von Kohlenwasserstoff zu halogeniertem Kohlenwasserstoff liegt dabei vorzugsweise im Bereich von 1:10 bis 10:1, besonders bevorzugt im Bereich von 4:1 bis 1:4 und insbesondere im Bereich von 2:1 bis 1:2.

In der Regel führt man die kationische Polymerisation bei Temperaturen unterhalb 0°C, z. B. im Bereich von 0 bis -140°C, vorzugsweise im Bereich von -30 bis -120°C, und besonders bevorzugt im Bereich von -40 bis -110°C durch. Der Reaktionsdruck ist von untergeordneter Bedeutung.

Die Abführung der Reaktionswärme erfolgt in üblicher Weise, beispielsweise durch durch Wandkühlung und/oder unter Ausnutzung einer Siedekühlung. Hier hat sich insbesondere die Verwendung von Ethen und/oder Mischungen von Ethen mit den oben als bevorzugt genannten Lösungsmitteln bewährt.

Zur Herstellung von Blockcopolymeren kann das distale Kettenende, d.h. das vom Initiator abgewandte Ende des erhaltenen Isobuten-Polymers, mit Comonomeren, wie den oben aufgeführten, z.B. Vinylaromaten umgesetzt werden. So kann man z.B. zuerst Isobuten homopolymerisieren und anschließend das Comonomer zusetzen. Das dabei neu entstehende Comonomer-stämmige reaktive Kettenende wird entweder desaktiviert oder nach einer der nachstehend beschriebenen Ausführungsformen unter Ausbildung einer funktionellen Endgruppe terminiert oder zur Bildung höherer Blockcopolymere erneut mit Isobuten umgesetzt.

Zum Reaktionsabbruch werden die lebenden Kettenenden desaktiviert, beispielsweise durch Zugabe einer protischen Verbindung, insbesondere durch Zugabe von Wasser, Alkoholen wie Methanol, Ethanol, n-Propanol, Isopropanol, n-Butanol, Isobutanol, sec.-Butanol oder tert.-Butanol, oder deren Mischungen mit Wasser.

Um di- oder trifunktionelle (telechele) Isobutenpolymere zu erhalten, terminiert man das distale Kettenende unter Ausbildung einer ethylenisch ungesättigten Gruppe, wobei man z. B. das reaktive Kettenende mit einem Terminierungsreagenz umsetzt, das an das Kettenende eine ethylenisch ungesättigte Gruppe anfügt, oder geeignet behandelt, um das reaktive Kettenende in eine solche Gruppe umzuwandeln.

In einer Ausführungsform wird das Kettenende durch Zugabe einer Trialkylallylsilanverbindung, z.B. Trimethylallylsilan, terminiert. Die Verwendung der Allylsilane führt zum Abbruch der Polymerisation unter Einführung eines Allylrestes am Polymerkettenende, vgl. EP 264 214.

In einer weiteren Ausführungsform wird das reaktive Kettenende thermisch, beispielsweise durch Erwärmen auf eine Temperatur von 70 bis 200°C, oder durch Behandlung mit einer Base in eine Methyliden-Doppelbindung überführt. Geeignete Basen sind z.B. Alkalimetallalkoxide, wie Natriummethanolat, Natriumethanolat und Kalium-tert-Butanolat, basisches Aluminiumoxid, Alkalimetallhydroxide, wie Natriumhydroxid, und tertiäre Amine, wie Pyridin oder Tributylamin, vgl. Kennedy et al., Polymer Bulletin 1985, 13, 435-439. Bevorzugt wird Natriumethanolat verwendet.

In einer weiteren Ausführungsform wird das reaktive Kettenende mit einem konjugierten Dien, wie Butadien, umgesetzt, vgl. DE-A 40 25 961.

In einer weiteren Ausführungsform werden durch Zusatz eines Kopplungsmittels zwei oder mehrere lebende Polymerketten gekoppelt. "Kopplung" bedeutet die Ausbildung von chemischen Bindungen zwischen den reaktiven Kettenenden, so dass zwei oder mehrere Polymerketten zu einem Molekül verbunden werden.

Geeignete Kopplungsmittel weisen beispielsweise wenigstens zwei allylständig zur gleichen oder verschiedenen Doppelbindungen angeordnete elektrofuge Austrittsgruppen, z.B. Trialkylsilylgruppen, auf, so dass sich das kationische Zentrum eines reaktiven Kettenendes in einer konzertierten Reaktion unter Abspaltung der Austrittsgruppe und Verschiebung der Doppelbindung anlagern kann. Andere Kopplungsmittel weisen wenigstens ein konjugiertes System auf, an das sich das kationische Zentrum eines reaktiven Kettenendes unter Ausbildung eines stabi lisierten Kations elektrophil addieren kann. Durch Abspaltung einer Austrittsgruppe, z.B. eines Protons, entsteht dann unter Rückbildung des konjugierten Systems eine stabile s-Bindung zu der Polymerkette. Mehrere dieser konjugierten Systeme können durch inerte Spacer miteinander verbunden sein.

Zu den geeigneten Kopplungsmitteln zählen:
(i) Verbindungen, die wenigstens zwei 5-gliedrige Heterocyclen mit einem unter Sauerstoff, Schwefel und Stickstoff ausgewählten Heteroatom aufweisen, z.B. organische Verbindungen, die wenigstens zwei Furanringe aufweisen, wie 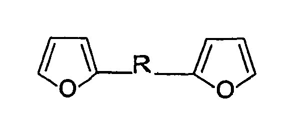 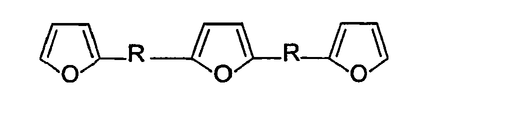 worin R für C₁-C₁₀-Alkylen steht, vorzugsweise Methylen oder 2,2-Propandiyl;
(ii) Verbindungen mit wenigstens zwei allylständigen Trialkylsilylgruppen, wie 1,1-Bis(trialkylsilylmethyl)ethylene, z.B. 1,1-Bis(trimethylsilylmethyl)ethylen,
   Bis[(trialkylsilyl)-propenyl]benzole z.B. 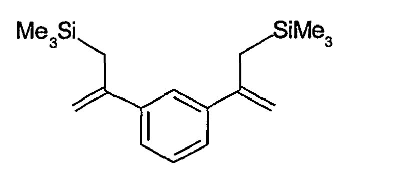 (worin Me für Methyl steht),
(iii) Verbindungen mit wenigstens zwei konjugiert zu jeweils zwei aromatischen Ringen angeordneten Vinylidengruppen, wie Bis-diphenylethylene z.B.

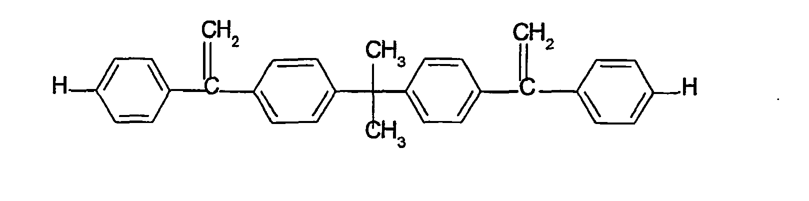

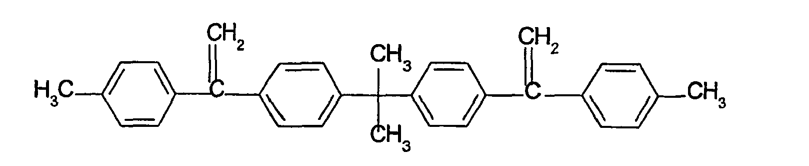

Eine Beschreibung geeigneter Kopplungsmittel findet sich in folgenden Literaturstellen; die Kopplungsreaktion kann in analoger Weise zu den dort beschriebenen Umsetzungen durchgeführt werden: R. Faust, S. Hadjikyriacou, Macromolecules 2000, 33, 730-733; R. Faust, S. Hadjikyriacou, Macromolecules 1999, 32, 6393-6399; R. Faust, S. Hadjikyriacou, Polym. Bull. 1999, 43, 121-128; R. Faust, Y. Bae, Macromolecules 1997, 30, 198; R. Faust, Y. Bae, Macromolecules 1998, 31, 2480; R. Storey, Maggio, Polymer Preprints 1998, 39, 327-328; WO99/24480; US 5,690,861 und US 5,981,785.

Die Kopplung erfolgt in der Regel in Gegenwart einer Lewis-Säure, wobei sich solche Lewis-Säuren eignen, die auch zur Durchführung der eigentlichen Polymerisationsreaktion verwendbar sind. Zur Durchführung der Kopplungsreaktion sind außerdem auch die gleichen Lösungsmittel und Temperaturen geeignet, wie man sie zur Durchführung der eigentlichen Polymerisationsreaktion einsetzt. Zweckmäßigerweise kann man die Kopplung daher als Eintopfreaktion im Anschluß an die Polymerisationsreaktion im gleichen Lösungsmittel in Gegenwart der zur Polymerisation eingesetzten Lewis-Säure durchführen. Üblicherweise verwendet man eine molare Menge des Kopplungsmittels, die etwa dem Quotienten der zur Polymerisation verwendeten molaren Menge des Initiators der Formel I, dividiert durch die Zahl der Kopplungsstellen des Kopplungsmittels, entspricht.

Nach der Terminierung oder Kopplung wird in der Regel das Lösungsmittel in geeigneten Aggregaten wie Rotations-, Fallfilm- oder Dünnschichtverdampfern oder durch Entspannung der Reaktionslösung entfernt.

Die nach dem erfindungsgemäßen Verfahren hergestellten Isobuten-Polymere weisen eine enge Molekulargewichtsverteilung auf. Der Polydispersitätsindex PDI = M_{w}/Mₙ liegt dabei vorzugsweise unterhalb von 1,40, besonders bevorzugt unterhalb von 1,35.

Die telechelen Polyisobutene können einer der folgenden Derivatisierungsreaktionen unterworfen werden:
Elektrophile Substitution an Aromaten

Das Polyisobuten kann in Gegenwart eines Alkylierungskatalysators mit einer (hetero)aromatischen Verbindung umgesetzt werden. Geeignete aromatische und heteroaromatische Verbindungen, Katalysatoren und Reaktionsbedingungen dieser sogenannten Friedel-Crafts-Alkylierung sind beispielsweise in J. March, Advanced Organic Chemistry, 4. Auflage, Verlag John Wiley & Sons, S. 534-539 beschrieben, worauf hier Bezug genommen wird.

Besonders eignen sich phenolische Verbindungen mit 1, 2 oder 3 OH-Gruppen, die gegebenenfalls wenigstens einen weiteren Substituenten aufweisen können. Bevorzugte weitere Substituenten sind Methyl und Ethyl. Besonders bevorzugt sind Phenol, die Kresol-Isomere, Katechol, Resorcinol, Pyrogallol, Fluoroglucinol und die Xylenol-Isomere. Insbesondere werden Phenol, o-Kresol und p-Kresol eingesetzt.

Geeignete Katalysatoren sind beispielsweise AlCl₃, AlBr₃, BF₃, BF₃.2 C₆H₅OH, BF₃[O(C₂H₅)₂]₂, TiCl₄, SnCl₄, AlC₂H₅Cl₂, FeCl₃, SbCl₅ und SbF₅. Diese Alkylierungskatalysatoren können gemeinsam mit einem Cokatalysator, beispielsweise einem Ether, wie Dimethylether, Diethylether, Di-n-propylether oder Tetrahydrofuran, eingesetzt werden. Die Reaktion kann auch mit Protonensäuren wie Schwefelsäure, Phosphorsäure, Trifluormethansulfonsäure katalysiert werden. Organische Protonensäuren können auch in polymer gebundener Form vorliegen, beispielsweise als lonenaustauscherharz. Geeignet sind auch Zeolithe sowie anorganische Polysäuren.

Die Alkylierung kann lösungsmittelfrei oder in einem Lösungsmittel durchgeführt werden. Geeignete Lösungsmittel sind beispielsweise n-Alkane und deren Gemische und Alkylaromaten, wie Toluol, Ethylbenzol und Xylol sowie halogenierte Abkömmlinge davon.

Zur weiteren' Funktionalisierung kann man das erhaltene Polyisobutenylphenol einer Umsetzung im Sinne einer Mannichreaktion mit wenigstens einem Aldehyd, beispielsweise Formaldehyd, und wenigstens einem Amin, das wenigstens eine primäre oder sekundäre Aminfunktion aufweist, unterziehen, wobei man eine mit Polyisobuten alkylierte und zusätzlich wenigstens teilweise aminoalkylierte Verbindung erhält. Es können auch Reaktions- und/oder Kondensationsprodukte von Aldehyd und/oder Amin eingesetzt werden. Die Herstellung solcher Verbindungen sind in WO 01/25 293 und WO 01/25 294 beschrieben, auf die hiermit im vollen Umfang Bezug genommen wird.

### Epoxidierung

Das Polyisobuten kann mit einer Peroxidverbindungen epoxidiert werden. Geeignete Verfahren zur Epoxidierung sind in J. March, Advanced Organic Chemistry, 4. Auflage, Verlag John Wiley & Sons, S. 826-829 beschrieben, worauf hier Bezug genommen wird. Vorzugsweise wird als Peroxidverbindung wenigstens eine Persäure, wie m-Chlorperbenzoesäure, Perameisensäure, Peressigsäure, Trifluorperessigsäure, Perbenzoesäure und 3,5-Dinitroperbenzoesäure eingesetzt. Die Herstellung der Persäuren kann in situ aus den entsprechenden Säuren und H₂O₂ gegebenenfalls in Gegenwart von Mineralsäuren erfolgen. Weitere geeignete Epoxidierungsreagenzien sind beispielsweise alkalisches Wasserstoffperoxid, molekularer Sauerstoff und Alkylperoxide, wie tert.-Butylhydroperoxid. Geeignete Lösungsmittel für die Epoxidierung sind beispielsweise übliche, nicht polare Lösungsmittel. Besonders geeignete Lösungsmittel sind Kohlenwasserstoffe wie Toluol, Xylol, Hexan oder Heptan. Das gebildete Epoxid kann anschließend ringöffnend mit Wasser, Säuren, Alkoholen, Thiolen oder primären oder sekundären Aminen umgesetzt werden, wobei man u.a. Diole, Glycolether, Glycolthioether und Amine erhält.

### Hydroborierung

Das Polyisobuten kann mit einem (gegebenenfalls in situ erzeugten) Boran umgesetzt werde, wobei ein wenigstens teilweise hydroxyliertes Polyisobuten erhalten wird. Geeignete Verfahren zur Hydroborierung sind in J. March, Advanced Organic Chemistry, 4. Auflage, Verlag John Wiley & Sons, S. 783-789 beschrieben, worauf hiermit Bezug genommen wird. Geeignete Hydroborierungsreagenzien sind beispielsweise Diboran, das in der Regel in situ durch Umsetzung von Natriumborhydrid mit BF₃-Etherat erzeugt wird, Diisamylboran (Bis-[3-methylbut-2-yl]boran), 1,1,2-Trimethylpropylboran, 9-Borbicyclo[3.3.1]nonan, Düsocamphenylboran, die durch Hydroborierung der entsprechenden Alkene mit Diboran erhältlich sind, Chlorboran-Dimethylsulfid, Alkyldichlorborane oder H₃B-N(C₂H₅)₂.

Üblicherweise werden die gebildeten Alkylborane nicht isoliert, sondern durch nachfolgende Umsetzung direkt in die Wertprodukte überführt. Eine sehr bedeutsame Umsetzung der Alkylborane ist die Reaktion mit alkalischen Wasserstoffperoxid unter Erhalt eines Alkohols, der vorzugsweise formal der Anti-Markovnikov-Hydratisierung des Alkens entspricht. Des Weiteren können die erhaltenen Alkylborane einer Umsetzung mit Brom in Gegenwart von Hydroxid-Ionen unter Erhalt des Bromids unterzogen werden.

### En-Reaktion

Das Polyisobuten kann mit wenigstens einem Alken, das eine elektrophil-substituierte Doppelbindung aufweist, in einer En-Reaktion umgesetzt werden (siehe z.B. DE-A 4 319 672 oder H. Mach und P. Rath in "Lubrication Science II (1999), S. 175-185, worauf vollinhaltlich Bezug genommen wird). Bei der En-Reaktion wird ein als En bezeichnetes Alken mit einem Allyl-ständigen Wasserstoffatom mit einem elektrophilen Alken, dem sogenannten Enophil, in einer pericyclischen Reaktion, umfassend eine Kohlenstoff-Kohlenstoff-Bindungsknüpfung, eine Doppelbindungsverschiebung und einen Wasserstofftransfer umgesetzt. Vorliegend reagiert das Polyisobuten als En. Geeignete Enophile sind Verbindungen, wie sie auch als Dienophile in der Diels-Alder-Reaktion eingesetzt werden. Bevorzugt wird als Enophil Maleinsäureanhydrid eingesetzt. Dabei resultieren wenigstens teilweise mit Bernsteinsäureanhydridgruppen (Succinanhydridgruppen) funktionalisierte Polyisobutene.

Das mit Bemsteinsäureanhydridgruppen derivatisierte Polyisobuten kann einer Folgereaktion unterzogen werden, die ausgewählt ist unter:
α) Umsetzung mit wenigstens einem Amin unter Erhalt eines wenigstens teilweise mit Succinimidgruppen und/oder Succinamidgruppen funktionalisierten Polyisobutens,
β) Umsetzung mit wenigstens einem Alkohol unter Erhalt eines wenigstens teilweise mit Succinestergruppen funktionalisierten Polyisobutens, und
γ) Umsetzung mit wenigstens einem Thiol unter Erhalt eines wenigstens teilweise mit Succinthioestergruppen funktionalisierten Polyisobutens.

Die nachfolgenden Beispiele sollen die Erfindung näher veranschaulichen.

### Beispiele

### Beispiel 1: Umsetzung von 1,5-Dimethylcycloocta-1,5-dien mit Chlorwasserstoff-Gas in Methylenchlorid

In einem 500 ml Vierhalskolben wurden 100 g (0,73 mol) 1,5-Dimethylcycloocta-1,5-dien in 200 ml Methylenchlorid vorgelegt. Unter Kühlung auf 0 bis 5 °C Innentemperatur wurden bei Normaldruck innerhalb von 4 Stunden 60 g (1,64 mol) Chlorwasserstoff eingeleitet. Anschließend wurde nicht umgesetzter Chlorwasserstoff durch Strippen mit Stickstoff entfernt. Danach wurde das Lösungsmittel unter verringertem Druck vollständig entfernt. Schließlich wurde das Produkt destillativ gereinigt (Siedepunkt: 85-88 °C bei 2 mbar) und man erhielt 137,1 g (90 % der Theorie) 1,5-Dichlor-1,5-dimethylcyclo-octan als farblose Flüssigkeit. ¹H-NMR (CDCl₃; 500 MHz): 2,40-2,25 (m); 2,20-1,95 (m); 1,8-1,7 (m); 1,7-1,5 (m). ¹³C{¹H}-NMR (CDCl₃; 400 MHz): 74,6 (s, 2C); 42,4 (s, 4C); 34,5 (s, 2C); 21,5 (s, 2C).

### Beispiel 2: Umsetzung von 1,5-Dimethylcycloocta-1,5-dien mit Chlorwasserstoff-Gas

In einem 2 I Vierhalskolben wurden 250 g (1,83 mol) 1,5-Dimethylcycloocta-1,5-dien vorgelegt. Unter Kühlung auf 0 bis 10 °C Innentemperatur wurden bei Normaldruck innerhalb von 8 Stunden 140 g (3,84 mol) Chlorwasserstoff eingeleitet. Anschließend wurde nicht umgesetzter Chlorwasserstoff im Vakuum entfernt und das Produkt destillativ gereinigt (Siedepunkt: 85-88 °C bei 2 mbar) und man erhielt 357,6 g (93 % der Theorie) 1,5-Dichlor-1,5-dimethylcyclooctan als farblose Flüssigkeit. ¹H-NMR (CDCl₃; 500 MHz): 2,40-2,25 (m); 2,20-1,95 (m); 1,8-1,7 (m); 1,7-1,5 (m). ¹³C{¹H}-NMR (CDCl₃; 400 MHz): 74,5 (s, 2C); 42,3 (s, 4C); 34,5 (s, 2C); 21,6 (s, 2C).

### Beipiel 3: Umsetzung eines Isomerengemischs aus 1,5-Dimethylcycloocta-1,5-dien (84 %) und 1,6-Dimethylcycloocta-1,6-dien (16 %) mit Chlorwasserstoff-Gas in Hexan

In einem 500 ml Vierhalskolben wurden 150 g (1,10 mol) des Isomerengemischs in 200 ml Hexan vorgelegt. Unter Kühlung auf 5 bis 10 °C Innentemperatur wurden bei Normaldruck innerhalb von 5,5 Stunden 79 g (2,16 mol) Chlorwasserstoff eingeleitet. Anschließend wurde nicht umgesetzter Chlorwasserstoff und das Lösungsmittel unter verringertem Druck vollständig entfernt. man erhielt 191,2 g (91 % der Theorie) des Gemischs aus 1,5-Dichlor-1,5-dimethylcyclooctan (A) und 1,4-Dichlor-1,4-dimethylcyclooctan (B) als farblose Flüssigkeit. Schließlich wurde das Produkt destillativ gereinigt (Siedepunkt: 83-88 °C bei 2 mbar). ¹H-NMR (CDCl₃; 500 MHz): 2,40-2,25 (m); 2,20-1,95 (m); 1,8-1,7 (m); 1,7-1,5 (m). ¹³C{¹H}-NMR (CDCl₃; 400 MHz): 74,6 (s, 2C, A); 74,5 (s, 2C, B); 42,4 (s, 4C, B); 42,3 (s, 4C, A); 34,7 (s, 2C, B); 34,5 (s, 2C, A); 21,6 (s, 2C, A); 20,9 (s, 2C, B).

### Beispiel 4: Umsetzung eines Isomerengemischs aus 1,5-Dimethylcycloocta-1,5-dien (84 %) und 1,6-Dimethylcycloocta-1,6-dien (16 %) mit Chlorwasserstoff-Gas

In einem Autoklaven wurden 380 g (2,79 mol) des Isomerengemischs vorgelegt. Innerhalb von 3 Stunden wurden 210 g (5,76 mol) Chlorwasserstoff derart eingeleitet, dass der Innendruck 5 bar betrug und die Innentemperatur 25 °C nicht überstieg. Anschließend wurde nicht umgesetzter Chlorwasserstoff durch Strippen mit Stickstoff entfernt. Schließlich wurde das Produkt destillativ gereinigt (Siedepunkt: 83-88 °C bei 2 mbar) und man erhielt 568,7 g (97 % der Theorie) des Gemischs aus 1,5-Dichlor-1,5-dimethylcyclooctan (A) und 1,4-Dichlor-1,4-dimethylcyclooctan (B) als farblose Flüssigkeit. ¹H-NMR (CDCl₃; 500 MHz): 2,40-2,25 (m); 2,20-1,95 (m); 1,8-1,7 (m); 1,7-1,5 (m). ¹³C{¹H}-NMR (CDCl₃; 400 MHz): 74,5 (s, 2C, A); 74,5 (s, 2C, B); 42,4 (s, 4C, B); 42,3 (s, 4C, A); 34,7 (s, 2C, B); 34,6 (s, 2C, A); 21,6 (s, 2C, A); 21,0 (s, 2C, B).

### Beispiel 5. Polymerisation eines Isobutenoligomeren mit mittlerem Molgewicht von 5000 g/mol

In einem mit trockenem Stickstoff inertisierten 21-Glaskolben wurden 300 ml n-Hexan vorgelegt, welches über Nacht über Molsieb (3Å) vorgetrocknet worden war. Anschließend wurden 300 ml Dichlormethan über einen mit Aluminumoxidkugeln gefüllten Tropftrichter zugegeben. Man versetzte die Lösung mit einer Spatelspitze Phenantrolin und kühlte auf - 40 °C. Bei dieser Temperatur wurde die vorhandene Restmenge an Wasser mit n-Butyllithium bis zur Braunfärbung austitriert. Nun erhitzte man das Gemisch und überführte die getrockneten Lösungsmittel über einen Teflonschlauch mit Jägerventil in den eigentlichen Reaktionskolben, der zuvor mit trockenem Stickstoff inertisiert worden war. Wiederum wurden die Lösungsmittel abgekühlt (- 70 °C). 400 ml Isobuten wurden gasförmig über Molsieb (3Å) geleitet, dabei getrocknet und in einen mit Trockeneis/Aceton-gekühlten Tropftrichter, der auf den Reaktionskolben gesteckt ist, einkondensiert und anschließend in den Reaktionskolben abgelassen. Bei - 70 °C wurden dann unter Rühren 1,475 g Phenyltriethoxysilan und 10,46g Initiator in den Reaktionskolben gegeben. Kurz darauf gab man 4,93g TiCl₄ zum Starten der Reaktion zu. Man beobachtete einen Temperaturanstieg auf - 50 °C. Die Reaktion wurde über 2 Stunden bei - 50 °C geführt und dann mit Ethanol abgebrochen. Danach wurde der Reaktionskolben auf Raumtemperatur erwärmt und der Inhalt in einem Schütteltrichter mit einem Liter entsalztem Wasser gewaschen. Nach der Phasentrennung verwarf man die wässrige Phase und wusch die organische Phase ein weiteres Mal mit einem Liter Wasser. Nach dem erneuten Abtrennen der wässrigen Phase wurde die organische Phase über Kieselgel abgesaugt und bei 180 °C und 3 mbar zur Trockene eingedampft. Es verblieb ein farbloses hochviskoses Polymer.

Analytik: GPC: Zur Durchführung der Gelpermeationschromatographie wurde eine Kombination zweier Styragelsäulen (1000 und 10000Å) verwendet. Die Kalibrierung erfolgte nach Isobutenstandard. Mn: 5700 g/mol, Mw: 10249 g/mol; D: 1,7; Mp: 63635 g/mol.

## Patentansprüche

1. Substituiertes Cycloalkan der Formel Ia, Ib, Ic: 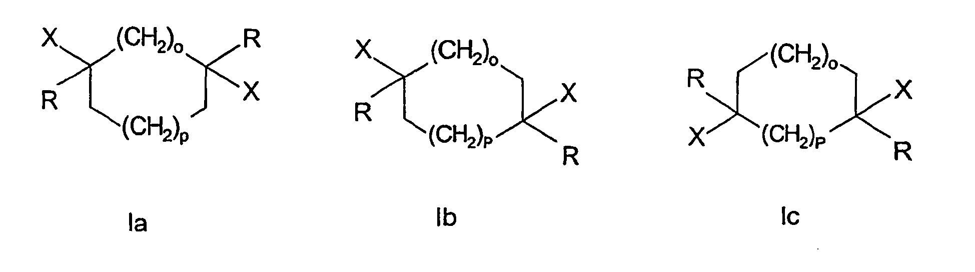 worin
R für C₁-C₆-Alkyl steht,
X für Halogen, OR¹ oder für OCOR¹ steht, worin R¹ für C₁-C₆-Alkyl steht,
o = 1 und p = 2, oder
o = 1 oder 2, p = 2 oder 3 und o+p = 4, oder
o = 1 oder 2, p = 3 oder 4 und o+p = 5.

2. Verbindung nach Anspruch 1, worin R für Methyl steht.

3. Verbindung nach Anspruch 1 oder 2, worin X für Chlor steht.

4. Verbindung nach Anspruch 1, ausgewählt unter 1,4-Dichlor-1,4-dimethylcyclo-octan, 1,5-Dichlor-1,5-dimethylcyclooctan und Gemischen davon.

5. Verfahren zur Herstellung eines substituieren Cycloalkans der Formel Ia, Ib oder Ic nach Anspruch 1, bei dem man ein Cycloalkapolyen der Formel IIa, IIb oder IIc 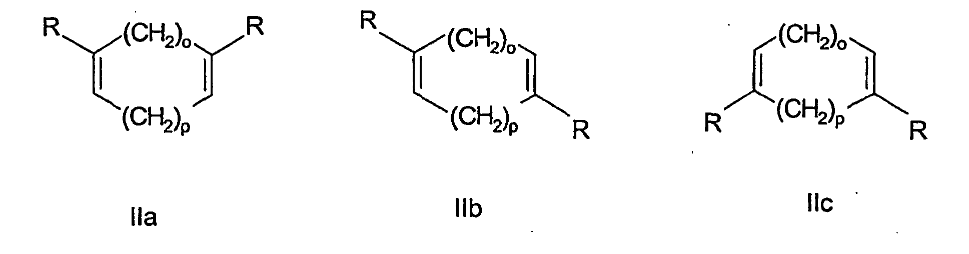 bei einer Temperatur von weniger als 40 °C mit einer Verbindung HX umsetzt, wobei die Symbole R, X, o und p die in Anspruch 1 angegebene Bedeutung haben.

6. Verfahren nach einem der Anspruch 5, wobei man als Verbindung HX gasförmigen Chlorwasserstoff verwendet.

7. Verfahren nach Anspruch 5 oder 6, wobei man als Cycloalkapolyen der Formel II 1,5-Dimethylcycloocta-1,5-dien und/oder 1,6-Dimethylcycloocta-1,5-dien verwendet.

8. Verfahren nach einem der Ansprüche 5 bis 7, wobei die Umsetzung in Abwesenheit eines Lösungsmittels oder Anwesenheit eines aprotischen Lösungsmittels erfolgt.

9. Verfahren der kationischen Polymerisation, bei dem man kationisch polymerisierbare ethylenisch ungesättigte Monomere in Gegenwart eines substituierten Cycloalkans der Formel I nach Anspruch 1 und einer Lewis-Säure polymerisiert.

10. Verfahren nach Anspruch 9, wobei es sich bei der Verbindung der Formel I um 1,5-Dichlor-1,5-dimethylcyclooctan und/oder 1,4-Dichlor-1,4-dimethylcyclooctan handelt.

11. Verfahren nach Anspruch 9 oder 10, wobei die kationisch polymerisierbaren ethylenisch ungesättigten Monomere Isobuten umfassen.

## Claims

1. A substituted cycloalkane of the formula Ia, Ib, Ic: 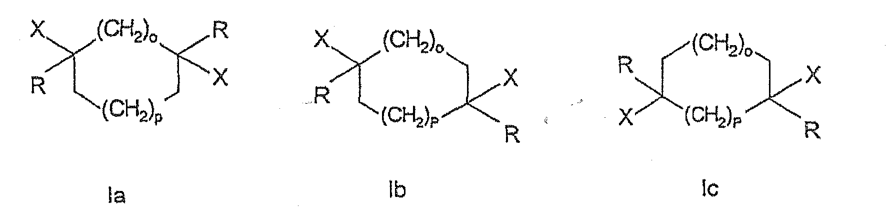 where
R is C₁-C₆-alkyl,
X is halogen, OR¹ or OCOR¹, where R¹ is C₁-C₆-alkyl,
o = 1 and p = 2, or
o=1 or 2, p = 2 or 3 and o+p = 4, or
o = 1 or 2, p = 3 or 4 and o+p = 5.

2. The compound according to claim 1 in which R is methyl.

3. The compound according to claim 1 or 2 in which X is chlorine.

4. The compound according to claim 1 selected from among 1,4-dichioro-1,4-dimethylcyclooctane, 1,5-dichloro-1,5-dimethylcyclooctane and mixtures thereof.

5. A process for preparing a substituted cycloalkane of the formula Ia, Ib or Ic according to claim 1, which comprises reacting a cycloalkapolyene of the formula Ila, IIb or IIc 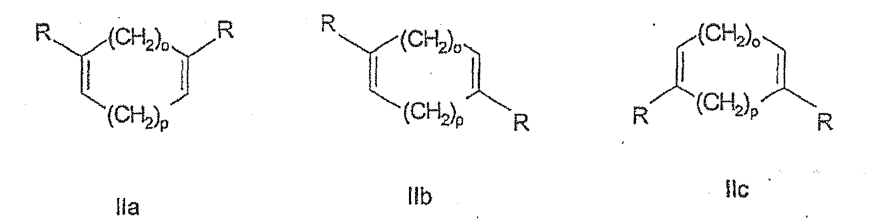 with a compound HX at below 40°C, where the symbols R, X, o and p are as defined in claim 1.

6. The process according to claim 5, wherein the compound HX used is gaseous hydrogen chloride.

7. The process according to claim 5 or 6, wherein the cycloalkapolyene of the formula II used is 1,5-dimethylcycloocta-1,5-diene and/or 1,6-dimethylcycloocta-1,5-diene.

8. The process according to any of claims 5 to 7, wherein the reaction is carried out in the absence of a solvent or in the presence of an aprotic solvent.

9. A cationic polymerization process which comprises polymerizing cationically polymerizable ethylenically unsaturated monomers in the presence of a substituted cycloalkane of the formula I according to claim 1 and a Lewis acid.

10. The process according to claim 9, wherein the compound of the formula I is 1,5-dichloro-1,5-dimethylcyclooctane and/or 1,4-dichloro-1,4-dimethylcyclooctane.

11. The process according to claim 9 or 10, wherein the cationically polymerizable ethylenically unsaturated monomers include isobutene.

## Revendications

1. Cycloalcane substitué de formule Ia, Ib, Ic : 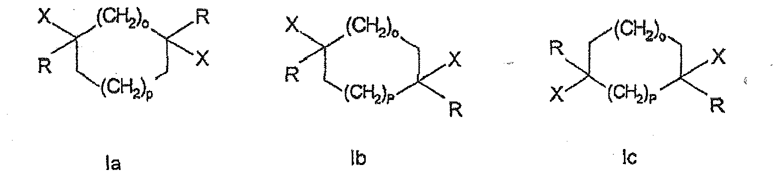 où
R représente un groupe alkyle en C₁-C₆,
X représente halogène, OR¹ ou OCOR¹, où R¹ représente C₁-C₆-alkyle,
o = 1 et p = 2, ou
O = 1 ou 2 , p = 2 ou 3 et o + p = 4 , ou
o = 1 ou 2 , p = 3 ou 4 et o + p = 5.

2. Composé selon la revendication 1, où R représente méthyle.

3. Composé selon la revendication 1 ou 2, où X représente chlore.

4. Composé selon la revendication 1, choisi parmi le 1,4-dichloro-1,4-diméthylcyclooctane, le 1,5-dichloro-1,5-diméthylcyclooctane et leurs mélanges.

5. Procédé pour la préparation d'un cycloalcane substitué de formule Ia, Ib ou Ic selon la revendication 1, dans lequel on transforme un cycloalcapolyène de formule IIa, IIb ou IIc 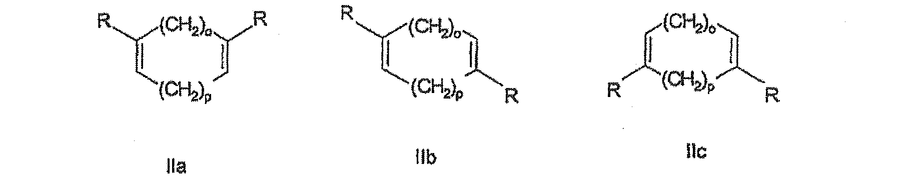 à une température inférieure à 40°C avec un composé HX, où les symboles R, X, o et p ont la signification indiquée dans la revendication 1.

6. Procédé selon la revendication 5, où on utilise comme composé HX du chlorure d'hydrogène gazeux.

7. Procédé selon la revendication 5 ou 6, où on utilise comme cycloalcapolyène de formule II du 1,5-diméthylcycloocta-1,5-diène et/ou du 1,6-diméthylcycloocta-1,5-diène.

8. Procédé selon l'une quelconque des revendications 5 à 7, où la transformation est réalisée en l'absence d'un solvant ou en l'absence d'un solvant aprotique.

9. Procédé de la polymérisation cationique, dans lequel on polymérise des monomères éthyléniquement insaturés polymérisables par voie cationique en présence d'un cycloalcane substitué de formule I selon la revendication 1 et d'un acide de Lewis.

10. Procédé selon la revendication 9, où il s'agit, pour le composé de formule I, de 1,5-dichloro-1,5-diméthylcyclooctane et/ou de 1,4-dichloro-1,4-diméthylcyclooctane.

11. Procédé selon la revendication 9 ou 10, où les monomères éthyléniquement insaturés polymérisables par voie cationique comprennent de l'isobutène.
